# EUROPEAN PATENT APPLICATION

(11) **EP 4 361 964 A1**
(43) Date of publication of application: **01.05.2024**
(21) Application number: 23201818.4
(22) Date of filing: 05.10.2023
(51) Int. Cl.: G06V 10/82, G06V 10/88, G06V 40/10

(54) **MUSCLE, SKIN OR BRAIN BASED AUTHENTICATION & IDENTIFICATION**

(30) Priority: 28.10.2022 US 202263420335 P; 08.03.2023 US 202318118833
(71) Applicant: Linear Dimensions Semiconductor Inc., Houston, TX 77036-3188 (US)
(72) Inventor: Schie, David, Houston (US)
(74) Representative: London IP Ltd

(57) **Abstract**

A device to extract a unique recognition vector from a user has a sensor generating an electrical signal responsive to one of: muscle, skin or brain activity of the user. A spectral conversion mechanism converts the electrical signal into a spectral image. A machine learning device converts the spectral image into a recognition vector associated with the user.

## Description

### TECHNICAL FIELD

The present application in general relates to an authentication or identification system, and more specifically, to an authentication or identification device for portable wearable devices having different form factors that is able to extract recognition vectors and compare these recognition vectors to identify or authenticate a person or animal.

### BACKGROUND

Currently, portable device authentication or identification (for example as used for cell phones or laptops) may be based upon recognition vectors generated by user actuated devices such as fingerprint sensors, facial identification (ID), eye scans or other biometric sensing devices. These types of devices generally require conscious interaction by the user to place their finger on a sensor, situate their face in front of the sensor, or provide a scan of their eye to use the authentication means.

For many devices, a means for authentication or identification of a user may not be compatible with current form factors. A new generation of devices is emerging for which these user actuated forms of authentication or identification may not be desirable. For example, emerging Augmented Reality/Virtual Reality (AR/VR) head mounted devices, glasses, ear buds, headsets, oral appliances, smart jewelry, eye masks, etc. do not work with facial ID because they are mounted on the head and cannot achieve a reasonable vantage point. These devices are not convenient for fingerprint sensors because it is difficult to locate the sensor by touch and inconvenient to do so. They are not convenient for retinal scans because they do not have access to the eye in certain cases such as ear buds.

Additionally, certain devices are not continuously secure because the user must initiate the authentication after which unlock might occur but not be verified. For example, a fork lift operator might authenticate with a local fingerprint sensor but then another driver might replace that operator and the machine may never know of the replacement.

In some wearable form factors, such as oral appliances for jaw and tooth adjustment or continuous positive airway pressure (CPAP) machines for sleep apnea, it is desired to have a means by which to ensure compliance or use of the device. For example, insurance companies may insist upon such compliance to pay for the equipment under certain health plans. Presently, temperature sensors may be used to ensure compliance. These temperature sensors maintain a data log of temperature and the result may be compared with the expected temperature of a human with variations over a period of time. Said temperature compliance, however, provides no evidence that the person using the device is the one for whom the device was intended and may be subject to tampering and error.

In commercial farming large animal herds may be common. It may be desirable to identify animals and separate them by a number of factors ranging from type of animal, to size, weight, sickness, or other characteristics. At present, branding or RF tags may be used. For example, RF tags may be attached to the ear of an animal. However, there currently is no specific biometric coupling to the animal so these tags can be switched between animals such that error, fraud or theft may be possible.

In biological systems, it may be common to introduce optical wavelengths such as infrared. For example, one may couple a light into the skin of a subject using an Infrared (IR) Light Emitting Diode (LED) or laser, and measuring the response or variation in a return signal. Another example may be by coupling an IR wavelength and measuring the response from many parts of the human body and provide pulse information (plethysmogram or ppg) as more or less light is absorbed by the underlying veins or arteries during or after the different phases of a heartbeat.

In certain industries where automotive uses may need to be monitored, such as, but not limited to, the use of a fork lift, other construction equipment, public transportation, first responders or other applications where the user is controlling or driving machinery, it may be dangerous for the user to initiate an authentication. At the same time, it may be desirable to ensure the user is the one authorized to use the equipment on a continuous or near continuous basis. It may also be desirable, to allow users to purchase items verbally from a car or other vehicle (point of purchase), to initiate start of the equipment without a key or fob, or to remember settings such as the seat or steering wheel position for each user of the equipment so the user does not have to readjust each time he enters the equipment after it was used by some other authorized user.

In AR/VR applications and glasses applications the eye movements of users may be unique. It is therefore possible to utilize movement of the eye where one or more of: i) eye movement; ii) eye focus response to light stimulus; iii) details of eye structure; iv) blinking patterns, etc. to create a recognition vector.

Therefore, it would be desirable to provide a device and method that overcomes the above. The device and method would provide a means of authentication or identification which would be compatible with desired form factors of the device being used.

### SUMMARY

In accordance with one embodiment, a device to extract a unique recognition vector from a user is disclosed. The device has a sensor generating an electrical signal responsive to one of: muscle, skin or brain activity of the user. A spectral conversion mechanism converts the electrical signal into a spectral image. A machine learning device converts the spectral image into a recognition vector associated with the user.

In accordance with one embodiment, a device to extract a unique recognition vector from a user is disclosed. The device has at least one sensor, wherein the at least one sensor is one of: an electrical sensor (capacitive or contact), an optical sensor, an ultrasonic sensor or an acoustic sensor coupled to a user, the at least one sensor providing an electrical signal responsive to optical, ultrasonic or acoustic activity variations extracted from optical, ultrasonic or acoustic inputs coupled to the user. A spectral conversion mechanism converts the electrical signal into a spectral image. A machine learning mechanism converts the spectral image into a recognition vector responsive to the user.

In accordance with one embodiment, a device to extract a unique recognition vector from a user is disclosed. The device has an image sensor watching an eye of the user. A machine learning device is coupled to the image sensor. The machine learning device measures at least one movement of the eye, pupil dilations, vibrations, blinking patterns and eye structure and distills the combination of one or more of those factors into a recognition vector responsive to the user.

### BRIEF DESCRIPTION OF THE DRAWINGS

The present application is further detailed with respect to the following drawings. These figures are not intended to limit the scope of the present invention but rather illustrate certain attributes thereof.
**FIG. 1** is a perspective view of an exemplary oral appliance having a device to extract a recognition vector in accordance with one aspect of the present application;
**FIG. 2** is a perspective view of an exemplary CPAP mask and tubing having a device to extract a recognition vector in accordance with one aspect of the present application;
**FIGs. 3A** - **3B** are illustrations of exemplary heart muscle waveforms which may be used by the device in accordance with one aspect of the present application;
**FIG. 4A** - **4B** are illustrations of an exemplary spectral images unique to the time frequency muscle response (wavelet) of two different people which may be used by the device in accordance with one aspect of the present application;
**FIG. 5** illustrates of an exemplary group of 128 byte recognition vectors in an identification database which may be used by the device in accordance with one aspect of the present application;
**FIG. 6** illustrates an exemplary growth of heart muscle packets or actuators which generate electromagnetic energy which can be plotted in three dimensions as illustrated by the 3d plot and which are unique for each individual and which may be used by the device in accordance with one aspect of the present application;
**FIGs. 7A - 7B** illustrates how the unique ridge and valley patterns of a fingerprint are analogous to the unique muscle packet emissions of a muscle in accordance with one aspect of the present application;
**FIG. 8A** illustrates a spectral image conforming to clean heart muscle inputs from a capacitive car seat sensor in accordance with one aspect of the present application;
**FIG. 8B** illustrates noise by static caused by the clothes and a sweater worn by the subject being measured in accordance with one aspect of the present application;
**FIGs. 9A** - **9E** illustrates exemplary embodiments of different glasses form factors in accordance with one aspect of the present application;
**FIG. 10A** - **10B** illustrates exemplary embodiments of an image sensor view of eyes from inside glasses or AR/VR form factors illustrating that blinking, pupil dilation and movement can be measured in accordance with one aspect of the present application;
**FIG. 11** shows an exemplary embodiment of ear buds having a sensor to extract a recognition vector in accordance with one aspect of the present application;
**FIG. 12** shows a car seat enabled by a device using a capacitive sensor which can extract muscle electrical information from a subject sitting on the car seat through clothes in accordance with one aspect of the present application;
**FIG. 13** shows different waveform responses (EOG) from capacitive sensor attached to eye wear placed near a user's eyes in accordance with one aspect of the present application;
**FIG. 14** illustrates an exemplary embodiment of clothing having a device to extract a recognition vector in accordance with one aspect of the present application;
**FIG. 15** illustrates an exemplary embodiment of headwear having a device to extract a recognition vector in accordance with one aspect of the present application;
**FIG. 16** illustrates an exemplary embodiment of jewelry having a device to extract a recognition vector in accordance with one aspect of the present application; and
**FIG. 17** illustrates an exemplary embodiment of a bock diagram of the device to extract a recognition vector in accordance with one aspect of the present application.

### DESCRIPTION OF THE APPLCIATION

The description set forth below in connection with the appended drawings is intended as a description of presently preferred embodiments of the disclosure and is not intended to represent the only forms in which the present disclosure can be constructed and/or utilized. The description sets forth the functions and the sequence of steps for constructing and operating the disclosure in connection with the illustrated embodiments. It is to be understood, however, that the same or equivalent functions and sequences can be accomplished by different embodiments that are also intended to be encompassed within the spirit and scope of this disclosure.

Embodiments of the exemplary device and method relates to an authentication or identification device for portable wearable devices having different form factors that is able to extracted recognition vectors and compare these recognition vectors to identify or authenticate a person or animal. The authentication or identification device may monitor and record electromagnetic energy radiated from muscles or optical variations measured from physical change of the subject which may then be converted into a spectral form, such as a wavelet image, which may then be analyzed by machine learning algorithms to produce a recognition vector responsive to the user and thereafter to compare the recognition vector to other such vectors stored in a database to identify or authenticate the user. The authentication or identification device may allow oral appliance and CPAP machines to authenticate or identify the user to ensure that tampering or fraud is not occurring. The authentication or identification device may be used to identify animals using ear tags or other biometric form factors so that there may be a true biometric identifier associated with the animal instead of just a number on a tag. The authentication or identification device may be used for other purposes as may be described below.

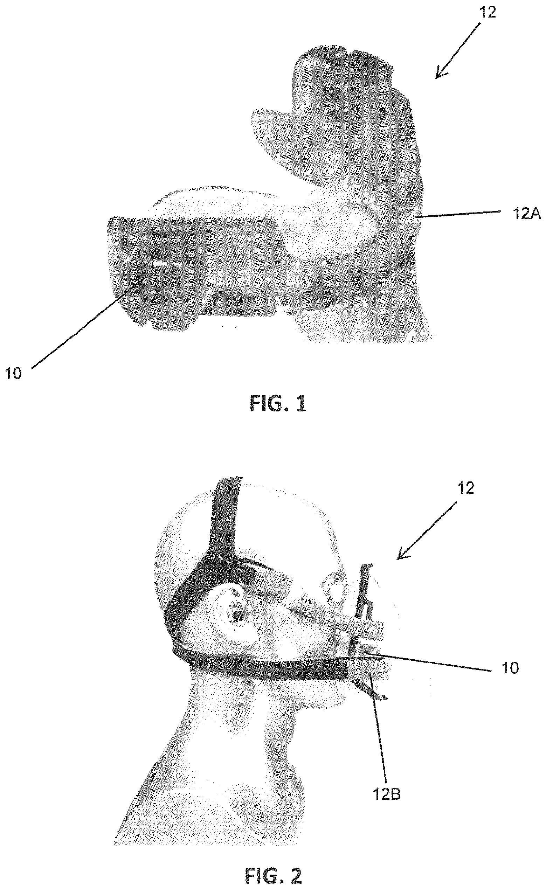

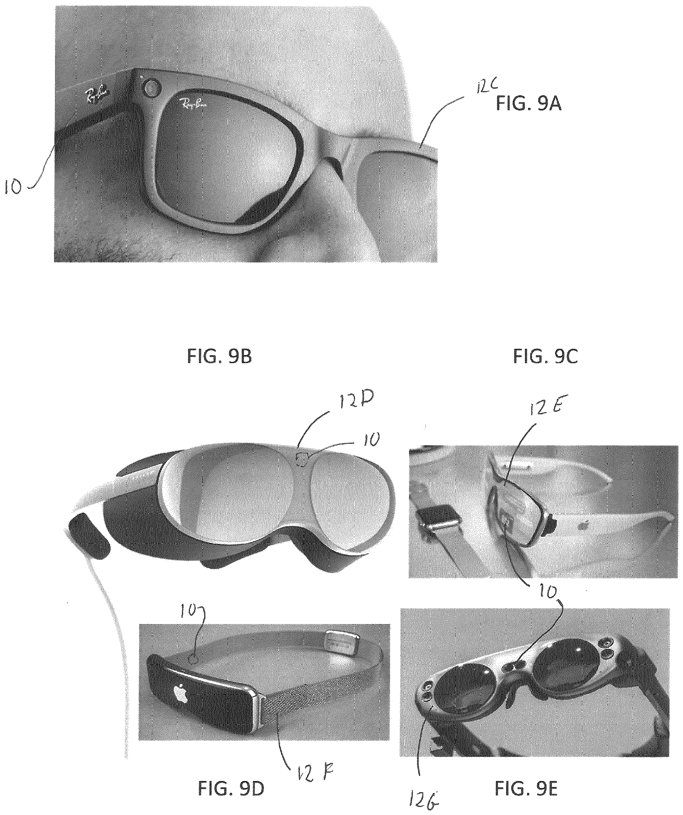

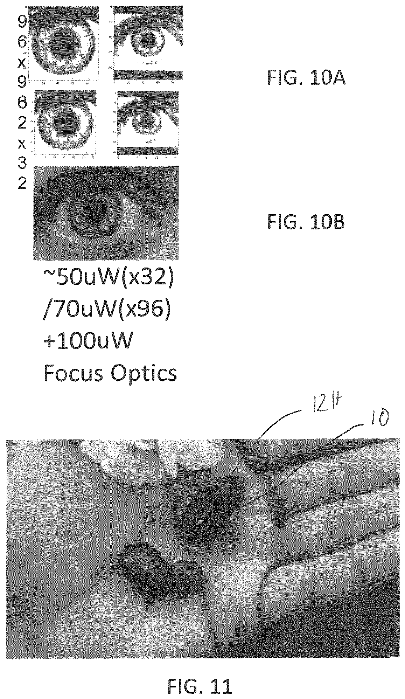

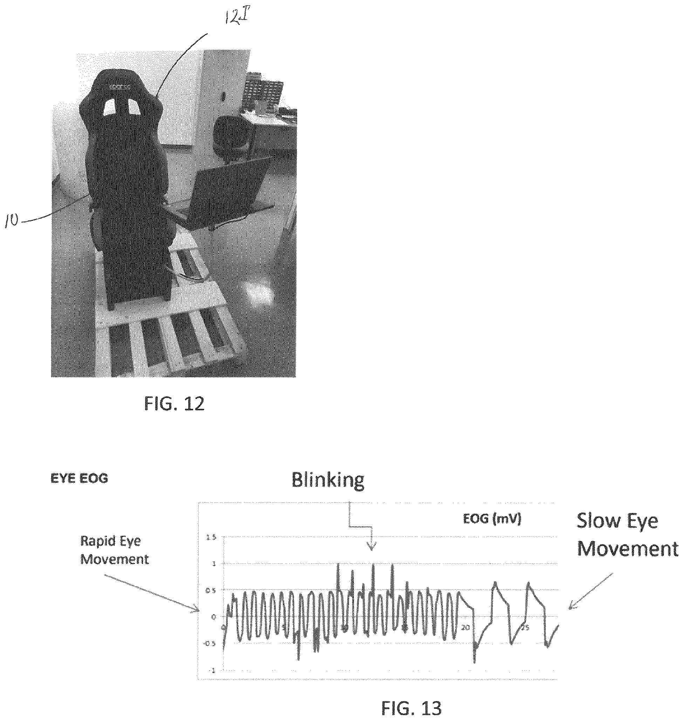

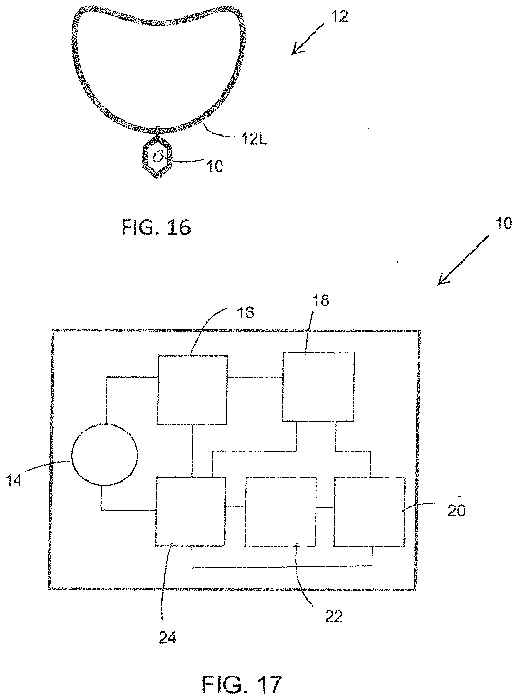

Referring to the FIGs., a device 10 for authentication or identification may be shown. The device 10 may be able to extract a unique recognition vector and compare the unique recognition vector to identify or authenticate a person or animal. The device 10 may be installed and used on portable wearable devices 12 having different form factors. For example, FIG. 1 shows that the device 10 may be positioned within an oral appliance 12A. The device 10 may be installed in a location that in the past may have been used for a temperature sensor. The temperature sensor may be used in past oral appliances 12A to monitor for compliance. The temperature sensor generally monitors the temperature to determine when the oral appliance 12A is being worn by the patient. In a similar manner, the device 10 may be used in a CPAP mask and tubing 12B as may be seen in FIG. 2 in order to determine when the appliance is being worn by the patient. FIGs. 9A-9E illustrates different eyewear which may use the device 10. FIG. 9A show a glasses 12C form factor with a camera mounted on the eye wear pointing outwards, but for which cameras can also be mounted internally to look at the eye, as well as other eyewear and AR/VR 12D-12G form factors which may use the device 10 which may use unique recognition vectors associated with eye biometrics such as, but not limited to: blinking, pupil dilation, eye movement and the like. The device 10 may also be used in ear buds 12H as may be seen in FIG. 11, a car seat 121 as shown in FIG. 12, or jewelry 12L as shown in FIG. 16. The above is given as examples and should not be seen in a limiting manner. The device 10 may be used with any wearable form factors, such as those mentioned above, as well as others such clothing, furniture, shoes and the like that do not work well with existing authentication and identification mechanisms such as face recognition, finger print sensors, iris sensors because they are hard to actuate by the user.

The device 10 may be compatible with battery constraints of the portable wearable device 12. For example, glasses 12C and AR/VR devices 12D are desirable if they are small and light. Notwithstanding the difficulty of scanning a face in these form factors, even the processors and mathematical engines required to do the image manipulation (ISP) and thereafter to distill a recognition vector requires significant power and therefore struggle in these form factors which have so little area and weight tolerance for batteries.

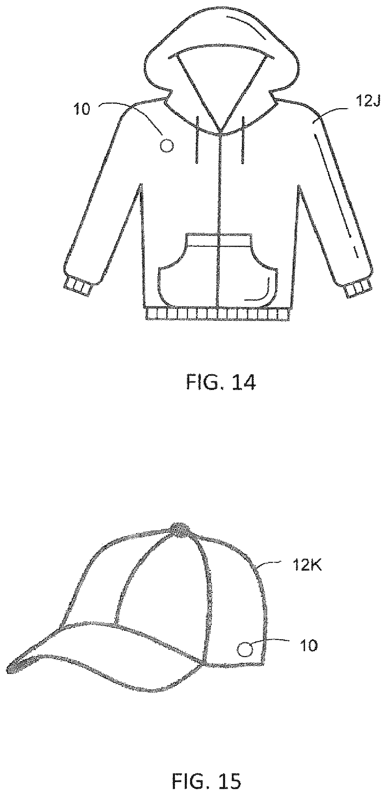

As may be seen in FIG. 14, the device 10 may have a sensor 14. The sensor 14 may generate an electrical signal responsive to the activity of one of: muscle, skin or brain of the user wearing the portable wearable devices 12 upon which the device 10 may be installed. When the portable wearable devices 12 is positioned on the user (i.e., a person or animal wearing the device 10), the sensor 14 may monitor for one of muscle, skin or brain activity/movement. For example, the sensor 14 may monitor for movement or activity of an eye, electrooculogram related muscles, jaw or mouth muscle, one or more electroencephalogram (EEG signals) from the brain, face muscles, forehead muscles, ear area muscles, neck muscles, heart muscles, arm muscles, hand muscles, finger muscles, stomach muscles, groin muscles, leg muscles, ankle muscles, foot muscles, toe muscles, galvanic skin response from anywhere on the skin and the like. Further, the sensor 14 may monitor a plethymogram or ppg optical return signal, a keytone variation signal, a glucagen variation signal, an ultrasonic return signal, an acoustic return signal, or the like.

The sensor 14 may be a capacitive sensor, a contact sensor, a field strength sensor, a magnetic sensor, a radar sensor, an ultrasonic capacitive micromachined ultrasonic transducer (CMUT) sensor, an acoustic Micro Electro-Mechanical (MEM) or piezo sensor, a silver-silver chloride sensor, a skin impedance sensor (also responsive galvanic skin response (GSR)), or other types of sensors.

The sensor 14 may be situated, mounted or coupled to portable wearable devices 12 and may depend on the form factors. For example, a capacitive sensor may be used in a car seat 12I, glasses 12C, or clothing such as a shirt, pants, jacket or headwear 12K to non-invasively extract signals of interest. In this example, the car seat 12I, or clothing 12J such as a shirt, pants or hat a copper cloth or conductive fiber may constituted one plate of the capacitive sensors and the user's body the other. In another example, a small CMOS image sensor may be used as the sensor 14 and positioned on the inside the corner or nose piece of the glasses 12C.

The device 10 may have a spectral conversion mechanism 16 coupled to the sensor 14. The spectral conversion mechanism 16 may convert the electrical signal generated by the sensor 12 into a spectral image. In accordance with one embodiment, spectral conversion may be a wavelet image. The spectral conversion mechanism 16 may also be an fft based time frequency analysis or other spectral mapping.

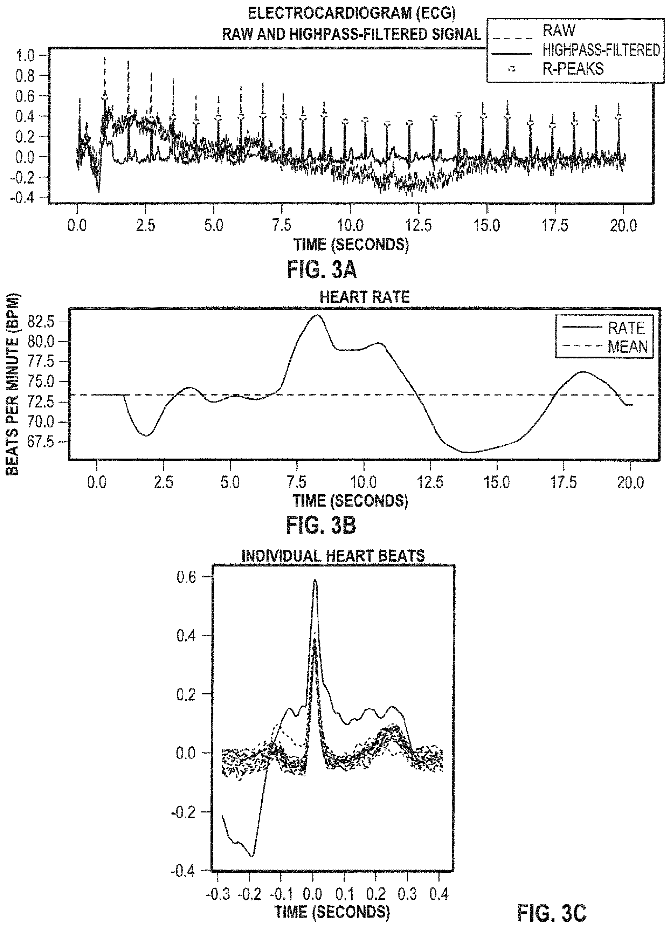

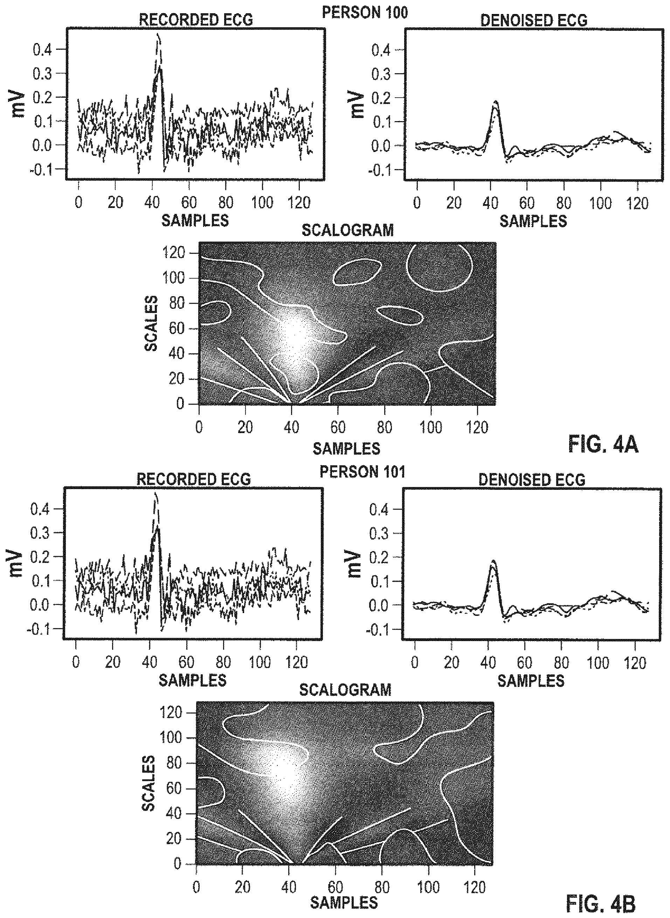

The spectral conversion mechanism 16 may create the spectral image based upon the frequency and time information contained within the electrical signal. For example, the sensor 14 may monitor the heart of the user. The electrical signals generated by the heart may be shown in different heart waveforms as may be shown FIGs. 3A-3C. In this example, the sensor 14 may record an electrocardiogram (EKG), heart rate and heartbeat of the heart. This data may be converted into spectral images as may be shown in FIGs. 4A-4B. The spectral images may be unique for different users.

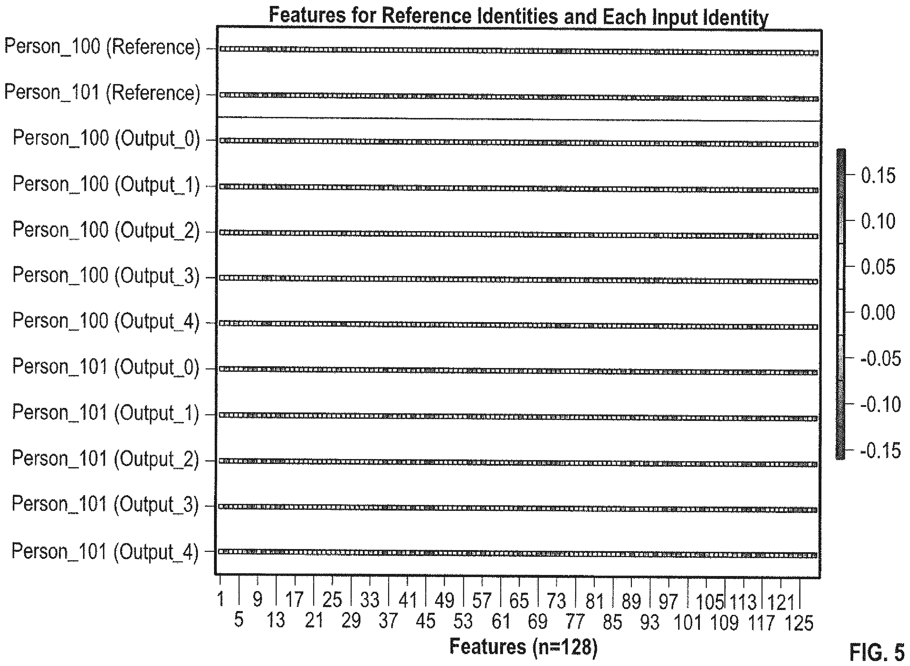

As stated above, since spectral images may be unique for different users, the recognition vectors for each user may also be unique. As may be shown in FIG. 5, a database of different recognition vectors for different users (i.e., people or animals) may be seen. As may be shown in FIG. 5, the spectral images for the same user as well as different users from various output locations may be shown. For example, reading may have been taken from a device 10 located in a mouth, heart, eye, and the like of the same user as well as different users. As may be seen, the spectral images may be unique for each output area for each user.

The spectral image may then be sent to a machine learning device 18. The machine learning device 18 may be a convolutional neural network, a fully connected network or the like. The machine learning device 18 may convert the spectral image into a recognition vector responsive to the user, i.e., a person or animal. The recognition vector formed may be a multidimensional recognition vector. Since the spectral images may be unique for each output area for each user, the recognition vector may also be unique for each output area of the user and may be used for identification and/or authorization purposes. Once an initial recognition vector has been formed based on a specific output area of a user, the recognition vector may be stored in a database 20. A comparison device 22 may be used to compare a currently extracted recognition vector from the same output to recognition vectors stored in the database 24 to identify or authenticate the user. In accordance with one embodiment, the device 10 may extract a recognition vector, such as a 128-byte recognition vector, such that the recognition vector could be compared to previously extracted recognition vectors stored in the database 20 so as to identify or authenticate a user. In accordance with one embodiment, the device 10 may initiate the extraction of the recognition vectors and thereafter the comparison to the comparison database 20, and could do so based on time or event based triggers. This may allow the device 10 to be a "continuous authenticator". In accordance with one embodiment, continuous authentication in automotive, construction equipment, may allow first responder or other users to concentrate on driving or operating the equipment while allow the users to interact to purchase, adjust settings or initiate operation of the vehicle/device.

The device 10 may have a power supply 22. The power supply 22 may be used to power each component of the device 10. In accordance with one embodiment, the power supply 22 may be a battery.

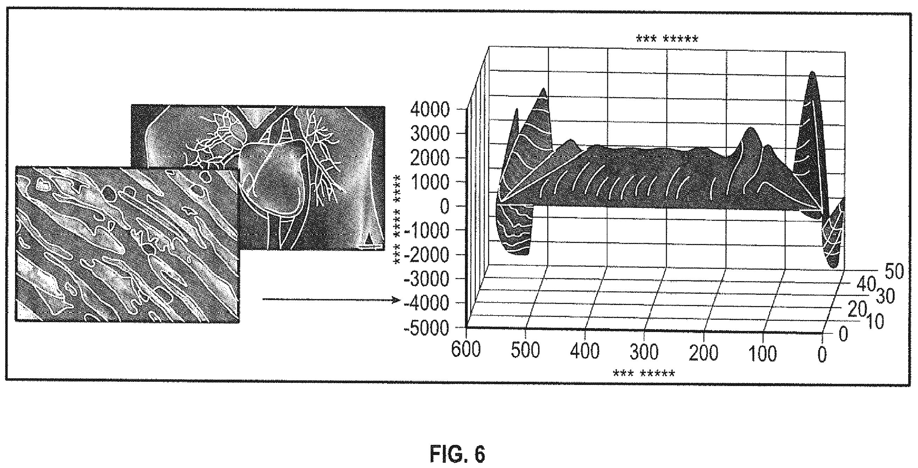

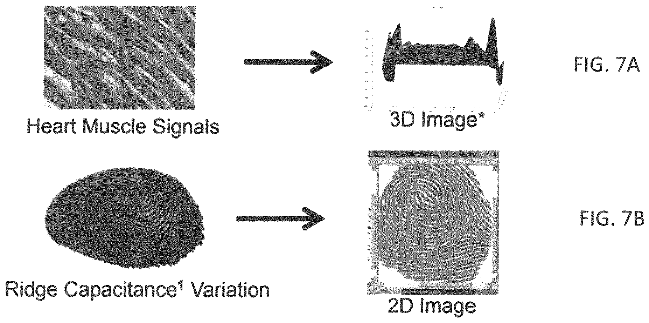

As stated above, the device 10 may be used on different parts of a user's body. FIG. 6 may show the growth of heart muscle packets or actuators which generate electromagnetic energy signals which can be plotted in three dimensions and which are unique for each individual. The uniqueness of three-dimensional plots formed by the muscle packet emissions may be similar to how the unique ridge and valley patterns of a fingerprint as may be seen in FIG. 7.

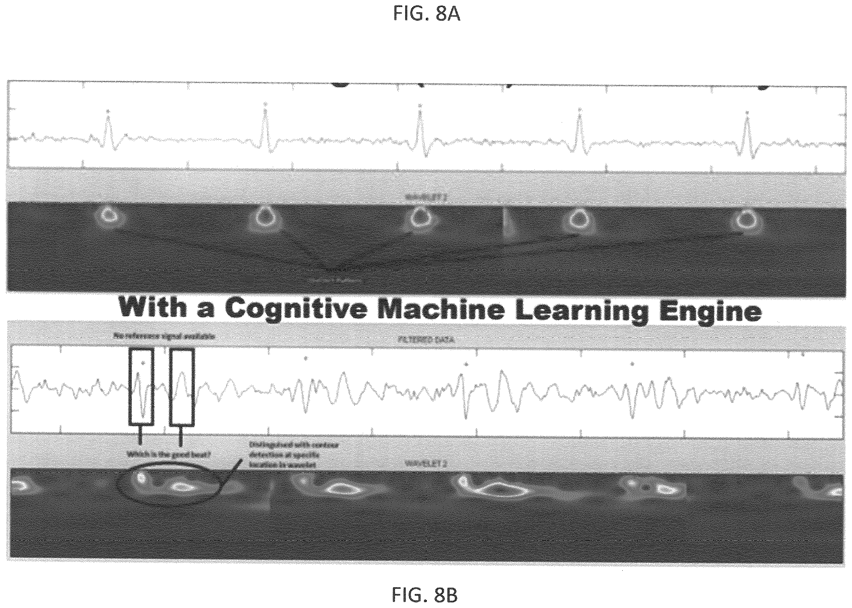

The device 10 may use machine learning in order to distinguish readings that may have been influenced by noise. For example, monitoring a user's heart muscle will differ when the device 10 may be placed directly on the skin versus a reading taken through clothing such as a shirt, sweater or the like. FIG. 8A-8B illustrates a spectral image conforming to clean (FIG. 8A) and noisy (FIG. 8B) heart muscle inputs. These reading may have been taken through a device 10 using a capacitive sensor which may have been installed in a car seat 12I. The second figure is noised by static caused by the clothes and/or sweater worn by the user being measured.

As previously stated, the device 10 may utilize movement of the eye where one or more of: eye movement, eye focus response to light stimulus, details of eye structure, blinking patterns, or similar eye may be used to create a recognition vector. FIG. 10 illustrates an image sensor view of eyes from inside glasses 12C or AR/VR form factors 12D-12G illustrating that blinking, pupil dilation and movement can be measured. FIG. 13 shows different waveform responses (EOG) from capacitive sensor attached to eye wear placed near a user's eyes.

While the above examples are showing the device being used to monitor heart and eye activity, the device 10 may be used to measure other muscles, skin or brain activity as disclosed above.

The device 10 may be configured for authentication or identification of users of portable wearable devices 12 having different form factors. The device 10 is able to extracte recognition vectors and compare these recognition vectors to identify or authenticate a person or animal. The device in 10 may comprise or be coupled to a wireless means such that the sensor output, spectral output or machine learning output may be coupled to an external device such as a cell phone, embedded system or external device. The device in 10 also may comprise or be coupled to a processor for performing said spectral conversion or machine learning. While certain examples are provided, it shall be clear to those skilled in the art that substitutions and variations to the taught means may be used while retaining the innovations taught in this application.

The foregoing description is illustrative of particular embodiments of the invention, but is not meant to be a limitation upon the practice thereof. The following claims, including all equivalents thereof, are intended to define the scope of the invention.

## Claims

1. A device to extract a unique recognition vector from a user comprising:
a sensor generating an electrical signal responsive to one of: muscle, skin or brain activity of the user;
a spectral conversion mechanism converting the electrical signal into a spectral image; and
a machine learning device converting the spectral image into a recognition vector associated with the user.

2. The device of Claim 1, wherein the sensor is at least one of: a capacitive sensor, a contact sensor, a field strength sensor, a magnetic sensor, a radar sensor, an ultrasonic CMUT sensor, an acoustic MEMs sensor, a piezo sensor, a silver-silver chloride sensor, a skin impedance sensor and the like.

3. The device of Claim 1 or 2, wherein the sensor monitors at least one of: eye muscles, electrooculogram related muscles, jaw muscles, mouth muscle, brain activity, electroencephalogram (EEG) signals from brain, facial muscles, forehead muscles, ear area muscles, neck muscles, heart muscles, arm muscles, hand muscles, finger muscles, stomach muscles, groin muscles, leg muscles, ankle muscles, foot muscles, toe muscles, galvanic skin response, and the like.

4. The device of any preceding Claim, wherein the device is coupled to eyewear, or is coupled to a headphone device, or is coupled to an article of clothing, or is coupled to headwear, or is coupled to an article of jewelry, or is coupled to an oral appliance.

5. The device of any of Claims 1 to 3, wherein the device is coupled to one of a CPAP mask or CPAP tubing.

6. The device of any of Claims 1 to 3, wherein the device is coupled to a seat.

7. The device of any preceding Claim wherein the recognition vector is used to adjust a position of a seat, or is used for point of purchase transactions.

8. The device of any preceding Claim, comprising a battery powering the device.

9. The device of any preceding Claim, wherein the spectral conversion mechanism is a wavelet image.

10. The device in accordance with any of Claims 1 to 8, wherein the spectral conversion mechanism is an fft based time frequency analysis.

11. The device of any preceding Claim, wherein the machine learning device is a convolutional neural network.

12. The device of any of Claims 1 to 10, wherein the machine learning device is a fully connected network.

13. The device of any preceding Claim, further comprising a database storing recognition vectors associated with the user.

14. The device of Claim 13, comprising a comparison device comparing a currently extracted recognition vector to recognition vectors stored in the database to authorize or identify the user.

15. The device of any preceding Claim, wherein the at least one sensor is responsive to at least one of: a plethymogram or ppg optical return signal, a keytone variation signal, a glucagen variation signal, an ultrasonic return signal, an acoustic return signal, or an image sensor watching an eye of the user.
